# EUROPEAN PATENT SPECIFICATION

(11) **EP 3 102 662 B1**
(45) Date of publication and mention of the grant of the patent: **28.07.2021**
(21) Application number: 15703557.7
(22) Date of filing: 04.02.2015
(51) Int. Cl.: C12G 3/025, C12G 3/024

(54) **PRODUCTION OF CIDER WITH PICHIA KLUYVERI YEAST**
HERSTELLUNG VON APFELWEIN MIT PICHIA-KLUYVERI-HEFE
PRODUCTION DE CIDRE AVEC LEVURE PICHIA KLUYVERI

(30) Priority: 04.02.2014 EP 14153806
(43) Date of publication of application: 14.12.2016
(73) Proprietor: Chr. Hansen A/S, 2970 Hoersholm (DK)
(72) Inventor: SAERENS, Sofie, 2740 Skovlunde (DK); SWIEGERS, Jan Hendrik, 3480 Fredensborg (DK)
(86) International application number: PCT/EP2015/052237
(87) International publication number: WO 2015/117978

(56) References cited:
- EP-A1- 2 641 962
- WO-A1-2009/110807
- WO-A1-2011/134952
- WO-A1-2013/030398
- WO-A2-2014/202564
- GB-A- 1 456 152
- KOMTHONG ET AL: "Effect of ascorbic acid on the odours of cloudy apple juice", FOOD CHEMISTRY, ELSEVIER LTD, NL, vol. 100, no. 4, 1 January 2007 (2007-01-01), pages 1342-1349, XP005589573, ISSN: 0308-8146, DOI: 10.1016/J.FOODCHEM.2005.10.070
- KAHLE K ET AL: "On-line gas chromatography combustion/pyrolysis isotope ratio mass spectrometry (HRGC-C/P-IRMS) of major volatiles from pear fruit (Pyrus communis) and pear products", FOOD CHEMISTRY, ELSEVIER LTD, NL, vol. 91, no. 3, 1 July 2005 (2005-07-01), pages 449-455, XP027770354, ISSN: 0308-8146 [retrieved on 2005-07-01]
- Sancho: "Infinite Dilution Activity Coefficients of Apple Juice Aroma Compounds", , 1 January 1997 (1997-01-01), pages 145-158, XP055117951, Retrieved from the Internet: URL:http://ac.els-cdn.com/S026087749789919 0/1-s2.0-S0260877497899190-main.pdf?_tid=e f686800-d9b7-11e3-a07b-00000aacb360&acdnat =1399887157_b1fe1563bab6ea9d72b1aa4a7319ff a5 [retrieved on 2014-05-14]
- PARK Y H ET AL: "Contribution a l'etude des levures de cognac. II. Etude des produits volatils formes au cours de la fermentation par les levures de cognac. [Study of cognac yeasts. II. Volatile compounds formed during fermentation by cognac yeasts.]", CONNAISSANCE DE LA VIGNE ET DU VIN, VIGNE ET VIN PUBLICATIONS INTERNATIONALES, BORDEAUX, FR, vol. 8, no. 4, 1 January 1974 (1974-01-01) , pages 343-373, XP008169264, ISSN: 0010-597X
- Valappil: "Impact of Thermal and Nonthermal Processing Technologies on Unfermented Apple Cider Aroma Volatiles", Journal of Agricultural and Food Chemistry, 11 February 2009 (2009-02-11), pages 924-929, XP055117375, Retrieved from the Internet: URL:http://pubs.acs.org/doi/pdf/10.1021/jf 803142d [retrieved on 2014-05-12]

## Description

### FIELD OF THE INVENTION

The present invention relates to the field of production of a beverage, especially cider, by fermentation of apple juice and/or pear juice. Specifically, the invention relates to a method of preparing a beverage with enhanced levels of desirable fermentation-derived flavor compounds comprising a step of fermentation of apple juice and/or pear juice with at least one *Pichia kluyveri* yeast strain, a beverage obtainable by the method and a cider comprising a high concentration of the flavor compound, hexyl acetate.

### BACKGROUND OF THE INVENTION

Aroma volatile compounds are important components of the flavor of fermented beverages, and exert an important influence on final consumer acceptance. In the case of cider, the aroma profile mainly depends on the apple variety used, but also on the choice of yeast used during fermentation. The aroma properties of the fruit used as raw material depend on the combination of volatile compounds present in the fruit, as well as on the concentration and flavor threshold of each volatile. Apple fruit produces many volatile compounds with different chemical structures. For most apple cultivars, esters with fruity notes are quantitatively and qualitatively predominant (Moya-Leon et al. 2007). Apple aroma increases with ripening and the time of harvest is a crucial factor in determining aroma production.

Different apple varieties have been analyzed for fruity ester volatile composition. In Royal Gala apples, butyl and hexyl acetate were the most abundant esters produced, and hexanol, butanol, and 2-methyl butanol were the most abundant higher alcohols found (Moya-Leon et al. 2007). Hexyl acetate, 2-methyl butyl acetate and butyl acetate were found to be major contributors to aroma. The concentrations of these volatiles were higher in late-harvested apples. The same observation was made for Fuji apples, where important contributors for apple aroma were methyl 2-methylbutanoate, isobutyl acetate, ethyl butanoate, butyl acetate and hexyl acetate (Komthong et al. 2007). In detail, Komthong et al. disclosed the effect of ascorbic acid on the odours of cloudy apple juice. Hexyl- and butyl acetates which have strong sweet fruity odours were the most prominent esters in the analyzed apple juice. The addition of ascorbid acid, used as antibrowning agent, did not have negative impact on the apple juice odour. In another apple variety, called Pink Lady, some of the same volatile ester compounds were measured during maturation: hexyl acetate, hexyl hexanoate, 2-methylbutyl acetate and butyl acetate (Villatoro et al. 2008). These aroma compounds were defined as the prominent aroma compounds that had the highest influence on differentiation of maturity stages. According to the authors, aroma volatile emission is an important factor for definition of fruit ripeness. This is confirmed in several recent articles, which report that ester production is a good indicator for ripeness of apples (Ortiz et al. 2011). The following esters seem the most important volatiles that define a "ripe" apple aroma: hexyl acetate, 2-methylbutyl acetate, hexyl hexanoate and isobutyl acetate.

In a sensory evaluation of character impact components in an apple model mixture, it was very clear that hexyl acetate and trans-2-hexenal are positive contributors that increase "apple" ratings, whereas hexanol is a negative contributor decreasing the "apple" ratings (Bult et al. 2002).

Several *Saccharomyces* species have been used to ferment apple juice and/or pear juice to cider: *Saccharomyces cerevisiae* and *Saccharomyces uvarum* (Leguerinel et al., 1988 & 1989; Scott and O'Reilly, 1996). However, no non*-Saccharomyces* strains have been used for production of apple cider so far.

Due to the increasing demand for healthier food and beverages, the reduction of ethanol in alcoholic beverages, such as cider, is of considerable commercial interest.

Low-alcohol or alcohol-free ciders are ciders with a low alcohol content or no alcohol which aim to reproduce the full flavor of standard ciders which normally contain at least 4.5% (vol/vol) alcohol.

Low-alcohol cider can be prepared by stopping fermentation by filtration or cooling and then maturation prior to bottling. Low-alcohol or non-alcohol ciders can also be made by producing full-strength cider with an alcohol concentration of above 4.5% (vol/vol) and then removing the alcohol by processes such as boiling off the alcohol or reverse osmosis. In some cases ciders with low alcohol contents are made simply by diluting full-strength ciders with water and/or apple/pear juice.
As these processes result in evaporation, loss or dilution of fermentation-derived flavor compounds, these types of ciders are often characterized as being less flavorful than full-strength cider or may contain high concentrations of undesirable flavors, such as hexanol which is present in different concentrations in apples of different varieties and maturation stage.

European patent application EP2641962 relates to the use of a *Pichia kluyveri* strain for fermentation of orange juice to produce a drink with a low alcohol content.
There is no mention of the use of *Pichia kluyveri* for fermentation of apple juice and/or pear juice.

WO2011/134952 discloses a method for inoculating a frozen yeast in juices. It suggests a number of applicable yeasts including *Pichia kluyveri, Saccharomyces cerevisiae, Saccharomyces pastorianus, Saccharomyces bayanus, Torulaspora delbreuckii,* and *Kluyveromyces thermotolerans.* A list of beverages is contemplated, including wine, beer, cider or other beverages which are produced by way of fermenting a fruit or vegetable material, such as fruit juice, vegetables, plants or fruit. Examples of fruit or vegetable material inlcude grapes, grape juice, apples or apple juice, or barley. However, WO2011/134952 does not explicitly disclose using *Pichia kluyveri* to prepare cider.

GB1456152 discloses an imitation apple flavour composition comprising a list of ingredients which includes hexyl acetate. This composition is diluted to prepare an apple beverage which contains 0.9 ppm hexyl acetate. As taught, adding ethyl safranate would make the flavoured beverage even more apple-like in odour and taste. The reference does not mention *Pichia kluyveri.*

There exists a need for alternative methods of producing beverages based on the fermentation of apple juice and/or pear juice wherein the presence of desirable flavor compounds, such as hexyl acetate and isobutyl acetate, is enhanced.

Furthermore, there exists a need for methods for preparing a low-alcohol or alcohol-free fermented beverage based on apple juice and/or pear juice with better organoleptic properties.

### SUMMARY OF THE INVENTION

It has unexpectedly been found that the fermentation of apple juice with *Pichia kluyveri* results in a very fresh cider product. Especially the main compounds regarded as giving "fresh apple flavor" were enhanced.

Since *Pichia kluyveri* consumes the sugar to produce flavor compounds with little ethanol produced, this method can be used to prepare non-alcohol or low-alcohol cider with increased amounts of hexyl acetate and other desirable flavor compounds.

When a higher percentage of alcohol is wanted, a longer fermentation with *Pichia kluyveri* and/or sequential fermentation with a *Saccharomyces* yeast can be performed.

The present invention provides use of at least one *Pichia kluyveri* strain for preparing a fermented beverage which is a cider having a hexyl acetate concentration of at least 0.7 ppm.

### BRIEF DESCRIPTION OF THE DRAWINGS

FIGURE 1 illustrates isobutyl acetate concentrations in 3 apple juices fermented with *Pichia kluyveri* strain PK-KR1 (juice with Pichia) or not fermented (con juice).
FIGURE 2 depicts isoamyl acetate concentrations in 3 apple juices fermented with *Pichia kluyveri* strain PK-KR1 (juice with Pichia) or not fermented (con juice).
FIGURE 3 shows ethyl propionate concentrations in 3 apple juices fermented with *Pichia kluyveri* strain PK-KR1 (juice with Pichia) or not fermented (con juice).
FIGURE 4 illustrates ethyl valerate concentrations in 3 apple juices fermented with *Pichia kluyveri* strain PK-KR1 (juice with Pichia) or not fermented (con juice).
FIGURE 5 depicts hexanol concentrations in 3 apple juices fermented with *Pichia kluyveri* strain PK-KR1 (juice with Pichia) or not fermented (con juice).
FIGURE 6 shows hexyl acetate concentrations in 3 apple juices fermented with *Pichia kluyveri* strain PK-KR1 (juice with Pichia) or not fermented (con juice).

### DETAILED DESCRIPTION OF THE INVENTION

The inventors of the present invention have unexpectedly found that *Pichia kluyveri* yeast strains, when used to ferment apple juice, are able to increase the presence of desirable flavor compounds in the fermented beverages. In particular, the flavor compound most associated with fresh apple flavor and aroma were found to be increased.

The process of preparing cider is well known to the skilled person and can be outlined in the following way; apple juice and/or pear juice is prepared by washing the fruits, optionally milling the fruits into pulp and extracting (preferably by pressing) the juice of the fruits without the pulp. Fermentation of the fruit juice is normally carried out either by spontaneous fermentation by indigenous yeast or by controlled fermentation in sterile settings by addition of cultured yeast but a combination of spontaneous fermentation and the addition of cultured yeast can also occur. After fermentation the cider is racked and the cider is optionally filled on bottles and optionally pasteurized.

During this process the individual cider maker may choose to exercise several other options, such as but not limited to the addition of pectinase during milling/pressing, adjustment of pH, addition of sulphur dioxide as an antimicrobial agent, malolactic fermentation, maturation in tanks or oak vats, fining and filtration, in order to achieve the desired end-product.

The term "cider" as used herein refers to the alcohol-free, low-alcohol or alcoholic beverage derived from apple juice (apple cider) or pear juice (perry cider) or blends of apple juice and pear juice by adding a yeast strain to ferment the apple juice and/or pear juice.

The term "alcohol-free beverage" herein is defined as a liquid for drinking with an alcohol content of no more than 0.5% (vol/vol) of alcohol.

The term "low-alcohol beverage" herein is defined as a liquid for drinking with an alcohol content of more than 0.5% (vol/vol) of alcohol and no more than 1.2% (vol/vol) of alcohol. The term "alcoholic beverage" herein is defined as a liquid for drinking with an alcohol content of more than 1.2% (vol/vol) of alcohol.

The method of preparing a beverage described herein comprises the steps of
a) providing an apple juice and/or pear juice; and
b) fermenting the apple juice and/or pear juice with at least one *Pichia kluyveri* yeast strain to obtain the beverage.

Preferably step b) comprises fermenting the apple juice and/or pear juice with at least one *Pichia kluyveri* yeast strain followed by fermenting the apple juice and/or pear juice with at least one *Saccharomyces* yeast strain.

*Saccharomyces* yeast strains include but is not limited to yeast of the species *Saccharomyces cerevisiae, Saccharomyces bayanus, Saccharomyces paradoxus, Saccharomyces pastorianus, Saccharomyces cariocanus, Saccharomyces kudriavzevii, Saccharomyces mikitae, Saccharomyces arboricolus* and *Saccharomyces uvarum.*

Preferably the *Saccharomyces* yeast strain is selected from the group consisting of a *Saccharomyces cerevisiae* yeast strain, a *Saccharomyces bayanus* yeast strain and a *Saccharomyces uvarum* yeast strain.

Preferably step b) comprises fermenting the apple juice and/or pear juice with at least one *Pichia kluyveri* yeast strain and allowing spontaneous fermentation of the apple juice and/or pear juice to take place.

The onset of spontaneous fermentation can readily be determined by the skilled person as CO₂ will start to form quickly and the concentration of ethanol will increase at a rate of at least 1% (vol/vol) per day.

Preferably, the fermentation of the apple juice and/or pear juice with the at least one *Pichia kluyveri* yeast strain is carried out for at least 12 hours, such as for at least 18 hours, such as for at least 24 hours, such as for at least 2 days, such as for at least 4 days, such as for at least 7 days.

Preferably the fermentation of the apple juice and/or pear juice with the at least one *Pichia kluyveri* yeast strain is carried out at a temperature of between 4-35°C.

More preferably the fermentation of the apple juice and/or pear juice with the at least one *Pichia kluyveri* yeast strain is carried out at a temperature of between 18-22°C.

Preferably the *Pichia kluyveri* yeast strain is inoculated in a concentration of at least 1x10⁴ CFU/ml, such as at least 5x10⁴ CFU/ml, such as at least 1x10⁵ CFU/ml, such as at least 5x10⁵ CFU/ml, such as at least 1x10⁶ CFU/ml, such as at least 5x10⁶ CFU/ml.

The described method preferably further comprises the step c) bottling and bottle pasteurizing the beverage.

Preferably the at least one *Pichia kluyveri* strain is selected from the group consisting of the *Pichia kluyveri* PK-KR1 (JT1.28 or strain A) strain that was deposited on 24 August 2006 at the National Measurement Institute, 541-65 Clarke Street, South Melbourne, Victoria 3205, Australia, by University of Auckland, School of Biological Sciences, Auckland 1142, New Zealand, under accession numbers V06/022711, and the *Pichia kluyveri* PK-KR2 (JT3.71) that was deposited on 24 August 2006 at the National Measurement Institute, 541-65 Clarke Street, South Melbourne, Victoria 3205, Australia, by University of Auckland, School of Biological Sciences, Auckland 1142, New Zealand, under accession numbers V06/022712.

As discussed herein and as exemplified in Example 1, the escribed herein method is very useful for preparing alcohol-free or low-alcohol beverages with increased levels of desirable fermentation-derived flavor compounds.

Thus, preferably, the beverage has an alcohol content of at the most 4.5% (vol/vol), such as at the most 1.2% (vol/vol).

Preferably, the fermentation is carried out in the absence of *Saccharomyces* yeast, such as with *Pichia kluyveri* yeast as the only yeast present.

Furthermore it is preferred that the fermentation of the apple juice and/or pear juice with the at least one *Pichia kluyveri* yeast strain is carried out for a maximum of 5 days.

The fermentation may be stopped by any suitable method known to the skilled person, including but not limited to chilling the beverage to a temperature below 4°C, filtration, pasteurization and centrifugation.

Alternatively, for producing an alcoholic beverage it is preferred to subsequently either perform controlled fermentation of the beverage with a *Saccharomyces* yeast strain or allow for spontaneous fermentation of the beverage (see Example 2).

Thus, preferably the method comprises a further step c) fermenting the apple juice and/or pear juice with a *Saccharomyces* yeast strain to obtain the beverage.

It is also described a beverage obtainable by the above method.

As can be seen from Examples 1 and 2 herein, the fermentation of apple juice with *Pichia kluyveri* increases significantly the amount of hexyl acetate in the fermented apple juice.

Thus, in a preferred embodiment the beverage has a hexyl acetate concentration of at least 0.7 ppm, such as at least 0.8 ppm, such as at least 0.9 ppm, such as at least 1.0 ppm, such as at least 1.1 ppm, such as at least 1.2 ppm, such as at least 1.3 ppm, such as at least 1.4 ppm, such as at least 1.5 ppm, such as at least 1.6 ppm, such as at least 1.7 ppm, such as at least 1.8 ppm, such as at least 1.9 ppm, such as at least 2.0 ppm.

Example 1 shows that fermentation of apple juice with Pichia kluyveri also increases significantly the amount of isobutyl acetate in the fermented apple juice.

Preferably, the beverage has an isobutyl acetate concentration of at least 0.1 ppm, such as at least 0.15 ppm, such as at least 0.2 ppm, such as at least 0.25 ppm.

It is also described a cider having a hexyl acetate concentration of at least 0.7 ppm, such as at least 0.8 ppm, such as at least 0.9 ppm, such as at least 1.0 ppm, such as at least 1.1 ppm, such as at least 1.2 ppm, such as at least 1.3 ppm, such as at least 1.4 ppm, such as at least 1.5 ppm, such as at least 1.6 ppm, such as at least 1.7 ppm, such as at least 1.8 ppm, such as at least 1.9 ppm, such as at least 2.0 ppm.

Preferably, the cider has an isobutyl acetate concentration of at least 0.1 ppm, such as at least 0.15 ppm, such as at least 0.2 ppm, such as at least 0.25 ppm.

More preferably, the cider has an alcohol concentration of at the most 4.5% (vol/vol), such as at the most 1.2% (vol/vol).

Preferably, the beverage is an apple cider.

### EXAMPLES

### Materials and methods

### Fermentation set-up

Lab-scale fermentation trials were carried out in 500 ml or 2 L of apple juice. Fermentation was performed for 24 hours or 7 days, as indicated, at 20°C. All yeast strains were inoculated at 5 million cells per ml.

### Headspace GC-FID analysis

Headspace gas chromatography coupled with flame ionization detection (GC-FID) was used for the measurement of esters and higher alcohols in the fermentation products. Fermentation samples were centrifuged, after which 2 ml was collected in vials. Samples were then analyzed with a calibrated Perkin Elmer GC System with a headspace sampler. The GC was equipped with a DB-WAXETR column (length, 30 m; internal diameter, 0.25 mm; layer thickness, 0.5 µm; Agilent Technologies, Germany). The split-splitless injector was used and held at 180°C. Samples were heated for 30 min at 70°C in the headspace autosampler before injection (needle temperature: 110°C). Helium was used as the carrier gas. After starting at 60°C, the oven temperature was raised after 2 min from 60°C to 230°C at 45°C/min and was finally held at 230°C for 5 min. During the GC-program a constant flow rate (10 mL/min) of the carrier gas (He) was maintained. The FID temperature was kept constant at 220°C respectively. The results were analyzed with Turbochrom software.

### Ethanol and sugar analysis

Ethanol and sugar analysis was carried out with Oenofoss™ equipment according to the manufacturer's protocol.

### Results

### Example 1. Production of alcohol-free cider with Pichia kluyveri.

Three different apple juices were fermented with a *Pichia kluyveri* yeast strain for the production of an alcohol-free cider:
- apple juice made from concentrate A
- apple juice made from concentrate B
- fresh apple juice

2 liter of apple juice was fermented with *Pichia kluyveri* strain PK-KR1 for 24 hours at 20°C. After 24 hours, samples were taken for flavor analysis to investigate the flavor profile of the fermented juices, compared with the control, where no yeast was added.

To measure how much sugar was degraded into ethanol, sugar and ethanol concentration was measured before and after fermentation (Table 1).

**Table 1. Sugar and ethanol levels of the different apple juices.**

| **Juice** | **Sugar level (°Brix) before fermentation** | **Sugar (°Brix) level after fermentation** | **Ethanol (% v/v) after fermentation** |
|---|---|---|---|
| Apple juice (concA) | 11.1 | 10.8 | 0.2 |
| Apple juice (concB) | 12.1 | 11.4 | 0.3 |
| Fresh apple juice | 12.2 | 11.8 | 0.2 |

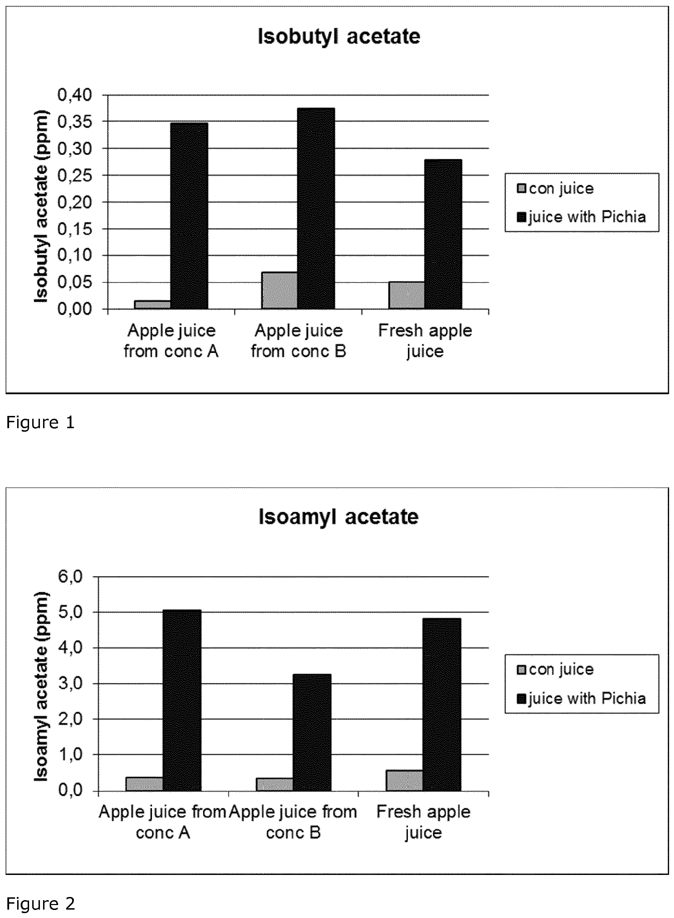

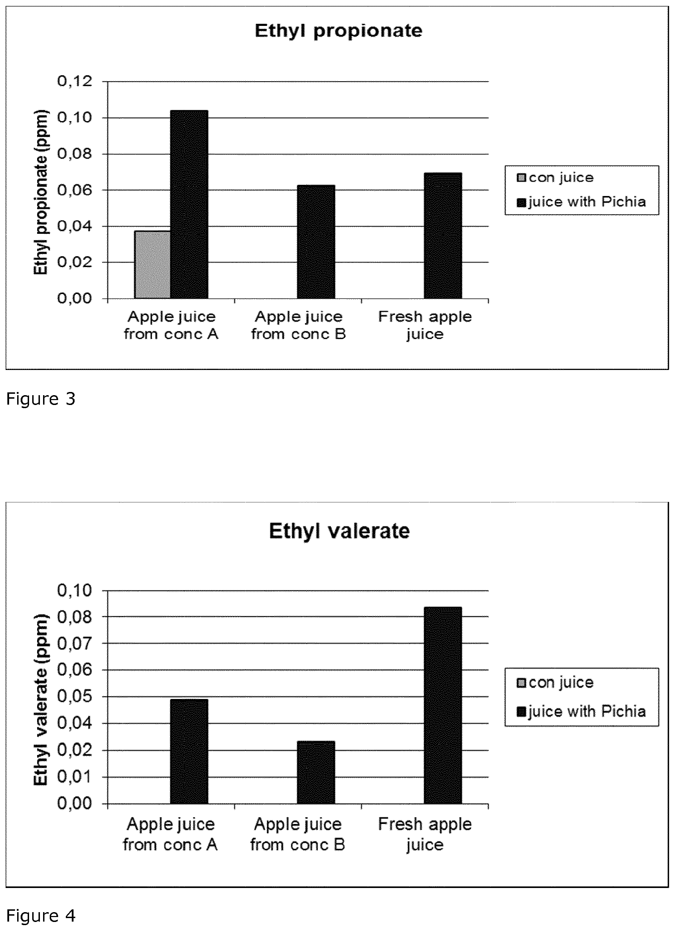

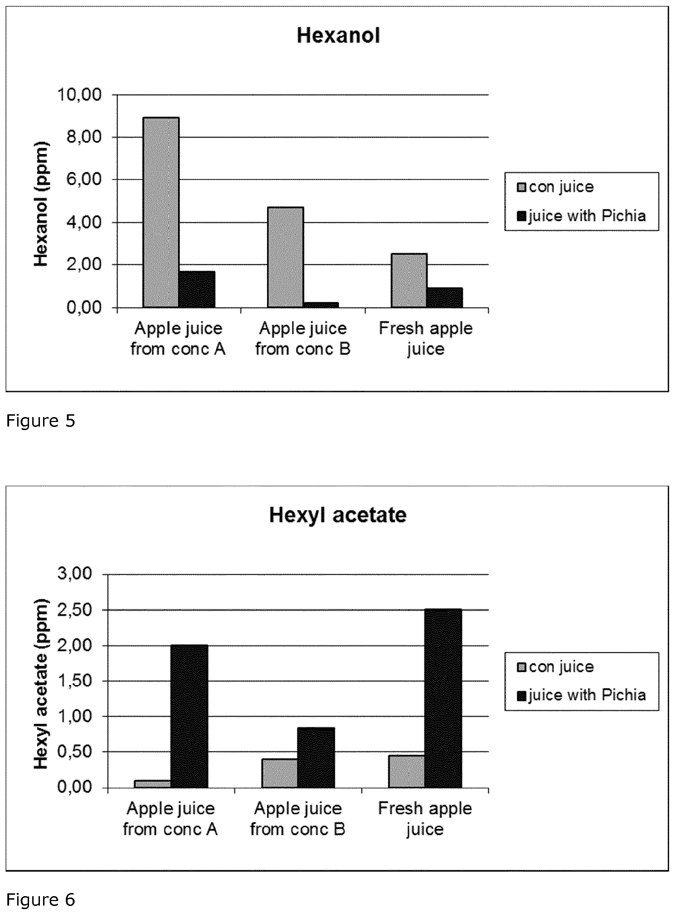

Flavor compounds were measured with headspace-GC-FID and the amounts of 6 compounds were found to be different (see Figures 1 to 6) in the fermented test juices as compared to the controls. These compounds were isobutyl acetate, isoamyl acetate, ethyl propionate, valerate, hexanol and hexyl acetate.
The amounts of ethyl butyrate and acetaldehyde did not significantly differ.

As can be seen from the results, adding *Pichia kluyveri* to the apple juice increased the production of isobutyl acetate, isoamyl acetate, ethyl propionate, ethyl valerate, and hexyl acetate. Hexanol is a precursor for hexyl acetate and this compound was very efficiently converted to hexyl acetate by the *Pichia kluyveri* strain, so we can see a clear decrease in hexanol and increase in hexyl acetate concentration.

Furthermore, *Pichia kluyveri* did not ferment a lot of sugar during the 24 hours, as only 0.2 to 0.3% of ethanol was formed. In this way, *Pichia kluyveri* seems to be an excellent yeast strain for fermenting juice without production of ethanol. Sensory analysis of the fermented apple juices revealed that the addition of *Pichia kluyveri* increased the fresh apple flavor of the apple juice and made the apple juice from concentrate smell like fresh apple juice. This means fresh apple flavor was enhanced in the apple juice from concentrate.

### Example 2. Production of alcoholic cider with Pichia kluvyeri, Torulaspora delbrueckii and Kluyveromyces thermotolerans.

To compare *Pichia kluyveri* with two other non*-Saccharomyces* yeast strains, *Torulaspora delbrueckii* and *Kluyveromyces thermotolerans,* cider from fresh apple juice was produced. In this experiment, the goal was to produce an alcoholic cider, as spontaneous fermentation was allowed to finish the fermentation (the apple juice was not pasteurized). The fresh apple juice was fermented at 20°C for 7 days and consisted of a mix of Belle de Boskoop, James Greaves, Cox Orange, Filipa and Cox Pomona apples. In all cases, a spontaneous fermentation started 2 days after addition of the non*-Saccharomyces* yeast strains (the rate of increase of the ethanol concentration reached at least 1% (vol/vol) per day) and finished the fermentation (Table 2).

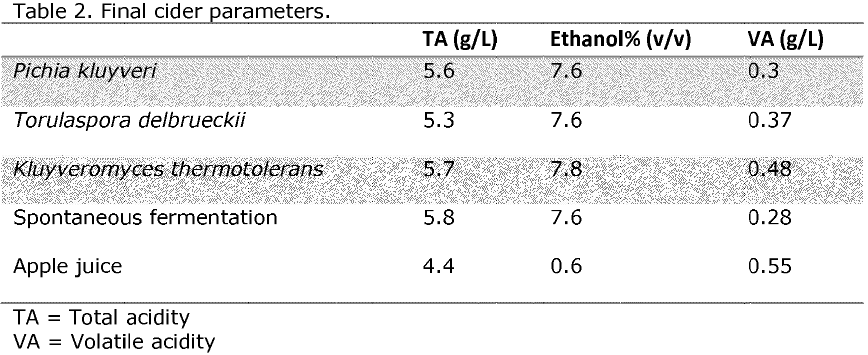

Final hexyl acetate and hexanol concentrations are given in Table 3.

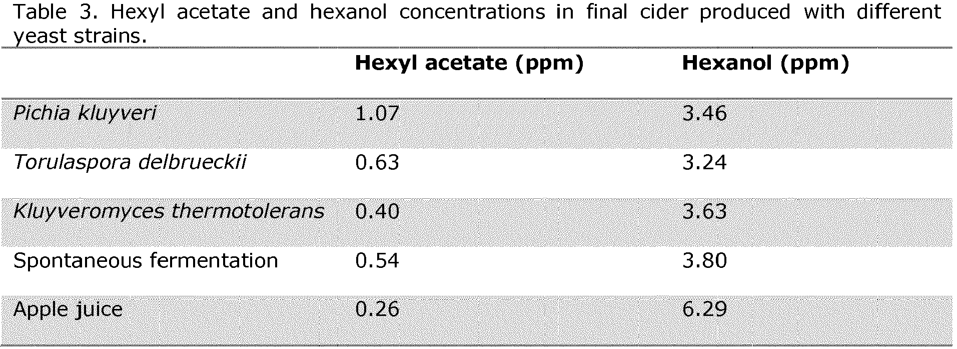

From Table 3, it is clear that the cider produced with *Pichia kluyveri* produces the highest amount of hexyl acetate, compared to the two other non-*Saccharomyces* strains. This means that using *Pichia kluyveri* for cider production is the best way to increase hexyl acetate in the final product. All yeast strains enhance the hexyl acetate concentration, compared to the basic apple juice, but *Pichia kluyveri* produces the highest amount in the final product. The hexanol concentration is more or less similar in all cider products, but the hexyl acetate/hexanol ratio is higher with *Pichia kluyveri,* resulting in an increased fresh apple flavor in the final product. Table 2 shows that all ciders have similar final ethanol concentrations, meaning that this technique can be used to produce extra flavor in an alcoholic cider as well. In this case, a sequential inoculation is needed with first a *Pichia kluyveri* yeast strain, followed by inoculation of a *Saccharomyces* sp. yeast strain or a spontaneous fermentation.

### Conclusion

It is very clear from the results that the use of *Pichia kluyveri* can enhance the fresh apple flavor in apple juice, making it suitable to use for both alcohol-free and alcoholic cider production. Especially the high production of hexyl acetate with *Pichia kluyveri* is very surprising. This compound is the most important flavor compound in cider determining fresh apple flavor. To produce this flavor compound, *Pichia kluyveri* uses hexanol as substrate, which is another very positive factor, as hexanol is regarded as a bad flavor compound in apple juice, resulting in a green astringent apple flavor and bad apple ratings.

*Pichia kluyveri* does not produce ethanol when it is fermented completely anaerobic, as the yeast is using all the sugar to produce flavor compounds. In this way, *Pichia kluyveri* can be used to produce alcohol-free cider, or can be combined with a *Saccharomyces* yeast to produce cider with fresh apple flavor.

### DEPOSITS

The *Pichia kluyveri* PK-KR1 (JT1.28 or strain A) and PK-KR2 (JT3.71) strains were deposited on 24 August 2006 at the National Measurement Institute, 51-65 Clarke Street, South Melbourne, Victoria 3205, Australia, by University of Auckland, School of Biological Sciences, Auckland 1142, New Zealand, and given the accession numbers V06/022711 and V06/022712 as described in WO 2009/110807.

### REFERENCES

Bult, J.H., Schifferstein, H.N., Roozen, J.P., Boronat, E.D., Voragen, A.D., and Kroeze, J.H. (2002) Sensory evaluation of character impact components in an apple model mixture. Chemical Senses, 27:485-94.
Komthong, P., Hayakawa, S., Katoh, T., Igura, N., and Shimoda, M. (2006) Determination of potent odorants in apple by headspace gas dilution analysis. In: LWT - Food Science and Technology, 39:472-478.
Leguerinel, I., Mafart, P., Cleret, J.J., and Bourgeois, C. (1988) Yeast strain and the formation of flavor components in cider. Journal of the Institute of Brewing, 96:391-395. Leguerinel, I., Mafart, P., Bourgeois, C. and Cleret, J.J. (1989) Yeast strain and kinetic aspects of the formation of flavour components in cider. Journal of the Institute of Brewing, 95:405-409.
Moya-Leon, M.A., Vergara, M., Bravo, C., Pereira, M. and Moggia C. (2007) Development of aroma compounds and sensory quality of 'Royal Gala' apples during storage. Journal of Horticultural Science and Biotechnology 82:403-413.
Ortiz, A., Graell, J., Lara, I. (2011) Volatile ester-synthesizing capacity throughout on-tree maturation of 'Golden Reinders' apples. Scientia Horticulturae, 131:6-14.
Villatoro, C., Altisent, R., Echeverria, G., Graell, J., Lopez, M .L., and Lara, I. (2008) Changes in biosynthesis of aroma volatile compounds during on-tree maturation of 'Pink Lady®' apples.

## Claims

1. Use of at least one *Pichia kluyveri* strain for preparing a fermented beverage which is a cider having a hexyl acetate concentration of at least 0.7 ppm.

2. Use according to claim 1 wherein the beverage has an alcohol concentration of at the most 1.2% (vol/vol) alcohol.

3. Use according to claim 1 wherein the beverage has an alcohol concentration of at the most 4.5% (vol/vol) alcohol.

4. Use according to any one of claims 1 to 3 wherein the cider is a pear cider.

5. Use according to any one of claims 1 to 3 wherein the cider is an apple cider.

6. Use according to any one of claims 1 to 5, wherein the fermented beverage is prepared by fermenting apple juice and/or pear juice for at least 12 hours with the at least one *Pichia kluyveri* yeast strain.

7. Use according to any one of claims 1 to 6, wherein the fermented beverage is prepared by fermenting the apple juice and/or pear juice with the at least one *Pichia kluyveri* yeast strain for a maximum of 5 days.

8. Use according to any one of claims 1 to 7 wherein the fermentation is carried out with *Pichia kluyveri* as the only yeast present.

9. Use according to any of claims 1 to 6 wherein spontaneous fermentation is allowed to take place in preparing the fermented beverage.

10. Use according to any of claims 1 to 6 wherein at least one *Saccharomyces* yeast strain is additionally used.

11. Use according to claim 10 wherein the *Saccharomyces* yeast strain is of the species *Saccharomyces cerevisiae, Saccharomyces bayanus, Saccharomyces paradoxus, Saccharomyces pastorianus, Saccharomyces cariocanus, Saccharomyces kudriavzevii, Saccharomyces mikitae, Saccharomyces arboricolus* or *Saccharomyces uvarum.*

12. Use according to any one of claims 1 to 11, wherein the at least one *Pichia kluyveri* strain is selected from the group consisting of the *Pichia kluyveri* PK-KR1 (JT1.28 or strain A) strain that was deposited on 24 August 2006 at the National Measurement Institute, 541-65 Clarke Street, South Melbourne, Victoria 3205, Australia, by University of Auckland, School of Biological Sciences, Auckland 1142, New Zealand, under accession numbers V06/022711, and the *Pichia kluyveri* PK-KR2 (JT3.71) that was deposited on 24 August 2006 at the National Measurement Institute, 541-65 Clarke Street, South Melbourne, Victoria 3205, Australia, by University of Auckland, School of Biological Sciences, Auckland 1142, New Zealand, under accession numbers V06/022712.

## Patentansprüche

1. Verwendung von mindestens einem *Pichia-kluyveri*-Stamm zur Herstellung eines fermentierten Getränks, das ein Cider mit einer Hexylacetat-Konzentration von mindestens 0,7 ppm ist.

2. Verwendung nach Anspruch 1, wobei das Getränk eine Alkoholkonzentration von höchstens 1,2 % (Vol./Vol.) Alkohol aufweist.

3. Verwendung nach Anspruch 1, wobei das Getränk eine Alkoholkonzentration von höchstens 4,5 % (Vol./Vol.) Alkohol aufweist.

4. Verwendung nach einem der Ansprüche 1 bis 3, wobei der Cider ein Birnen-Cider ist.

5. Verwendung nach einem der Ansprüche 1 bis 3, wobei der Cider ein Apfel-Cider ist.

6. Verwendung nach einem der Ansprüche 1 bis 5, wobei das fermentierte Getränk durch mindestens 12-stündiges Fermentieren von Apfelsaft und/oder Birnensaft mit dem mindestens einen Hefestamm *Pichia kluyveri* hergestellt wird.

7. Verwendung nach einem der Ansprüche 1 bis 6, wobei das fermentierte Getränk durch höchstens 5 Tage langes Fermentieren von Apfelsaft und/oder Birnensaft mit dem mindestens einen Hefestamm *Pichia kluyveri* hergestellt wird.

8. Verwendung nach einem der Ansprüche 1 bis 7, wobei die Fermentation mit *Pichia kluyveri* als einziger vorhandener Hefe durchgeführt wird.

9. Verwendung nach einem der Ansprüche 1 bis 6, wobei zugelassen wird, dass eine spontane Fermentation bei der Herstellung des fermentierten Getränks stattfindet.

10. Verwendung nach einem der Ansprüche 1 bis 6, wobei zusätzlich mindestens ein *Saccharomyces-*Hefestamm verwendet wird.

11. Verwendung nach Anspruch 10, wobei der *Saccharomyces-*Hefestamm der Spezies *Saccharomyces cerevisiae, Saccharomyces bayanus, Saccharomyces paradoxus, Saccharomyces pastorianus, Saccharomyces cariocanus, Saccharomyces kudriavzevii, Saccharomyces mikitae, Saccharomyces arboricolus* oder *Saccharomyces uvarum* angehört.

12. Verwendung nach einem der Ansprüche 1 bis 11, wobei der mindestens eine *Pichia-kluyveri*-Stamm aus der Gruppe bestehend aus dem Stamm *Pichia kluyveri* PK-KR1 (JT1.28 oder Stamm A), der am 24. August 2006 beim National Measurement Institute, 541-65 Clarke Street, South Melbourne, Victoria 3205, Australien, von der University of Auckland, School of Biological Sciences, Auckland 1142, Neuseeland, unter der Zugangsnummer V06/022711 hinterlegt wurde, und *Pichia kluyveri* PK-KR2 (JT3.71), der am 24. August 2006 beim National Measurement Institute, 541-65 Clarke Street, South Melbourne, Victoria 3205, Australien, von der University of Auckland, School of Biological Sciences, Auckland 1142, Neuseeland, unter der Zugangsnummer V06/022712 hinterlegt wurde, ausgewählt ist.

## Revendications

1. Utilisation d'au moins une souche de *Pichia kluyveri* pour préparer une boisson fermentée qui est un cidre ayant une concentration en acétate d'hexyle d'au moins 0,7 ppm.

2. Utilisation selon la revendication 1, dans laquelle la boisson a une concentration en alcool d'au plus 1,2 % (vol/vol) d'alcool.

3. Utilisation selon la revendication 1, dans laquelle la boisson a une concentration en alcool d'au plus 4,5 % (vol/vol) d'alcool.

4. Utilisation selon l'une quelconque des revendications 1 à 3, dans laquelle le cidre est un cidre de poire.

5. Utilisation selon l'une quelconque des revendications 1 à 3, dans laquelle le cidre est un cidre de pomme.

6. Utilisation selon l'une quelconque des revendications 1 à 5, dans laquelle la boisson fermentée est préparée en faisant fermenter du jus de pomme et/ou du jus de poire pendant au moins 12 heures avec l'au moins une souche de levure *Pichia kluyveri.*

7. Utilisation selon l'une quelconque des revendications 1 à 6, dans laquelle la boisson fermentée est préparée en faisant fermenter le jus de pomme et/ou le jus de poire avec l'au moins une souche de levure *Pichia kluyveri* pendant un maximum de 5 jours.

8. Utilisation selon l'une quelconque des revendications 1 à 7, dans laquelle la fermentation est effectuée avec *Pichia kluyveri* comme seule levure présente.

9. Utilisation selon l'une quelconque des revendications 1 à 6, dans laquelle la fermentation spontanée peut avoir lieu lors de la préparation de la boisson fermentée.

10. Utilisation selon l'une quelconque des revendications 1 à 6, dans laquelle au moins une souche de levure *Saccharomyces* est en outre utilisée.

11. Utilisation selon la revendication 10, dans laquelle la souche de levure *Saccharomyces* appartient aux espèces *Saccharomyces cerevisiae, Saccharomyces bayanus, Saccharomyces paradoxus, Saccharomyces pastorianus, Saccharomyces cariocanus, Saccharomyces kudriavzevii, Saccharomyces mikitae, Saccharomyces arboricolus* ou *Saccharomyces uvarum.*

12. Utilisation selon l'une quelconque des revendications 1 à 11, dans laquelle l'au moins une souche *Pichia kluyveri* est choisie dans le groupe constitué de la souche *Pichia kluyveri* PK-KR1 (JT1.28 ou souche A) qui a été déposée le 24 août 2006 au National Measurement Institute, 541-65 Clarke Street, South Melbourne, Victoria 3205, Australie, par l'Université d'Auckland, School of Biological Sciences, Auckland 1142, Nouvelle-Zélande, sous les numéros d'accession V06/022711, et *Pichia kluyveri* PK-KR2 (JT3.71) qui a été déposée le 24 août 2006 au National Measurement Institute, 541-65 Clarke Street, South Melbourne, Victoria 3205, Australie, par l'Université d'Auckland, School of Biological Sciences, Auckland 1142, Nouvelle-Zélande, sous les numéros d'accession V06/022712.
